# EUROPEAN PATENT SPECIFICATION

(11) **EP 3 087 931 B1**
(45) Date of publication and mention of the grant of the patent: **03.04.2019**
(21) Application number: 16167433.8
(22) Date of filing: 28.04.2016
(51) Int. Cl.: A61B 17/072, A61B 17/34, A61B 17/00, A61B 90/00

(54) **BUTTRESS BRACHYTHERAPY AND INTEGRATED STAPLE LINE MARKERS FOR MARGIN IDENTIFICATION**
STÜTZ-BRACHYTHERAPIE UND INTEGRIERTE KLAMMERREIHENMARKER ZUR RANDIDENTIFIZIERUNG
CURIETHERAPIE A CONTREFORT ET MARQUEURS DE LIGNE A AGRAFE INTEGREE POUR IDENTIFICATION DE MARGE

(30) Priority: 29.04.2015 US 201514699179
(43) Date of publication of application: 02.11.2016
(73) Proprietor: Covidien LP, Mansfield, MA 02048 (US)
(72) Inventor: GLEIMAN, Seth, Guilford, CT 06437 (US); RACENET, David, Killingworth, CT 06419 (US); HODGKINSON, Gerald, Guilford, CT 06437 (US)
(74) Representative: Soames, Candida Jane

(56) References cited:
- EP-A1- 2 305 135
- EP-A1- 2 481 359
- EP-A2- 1 815 804
- WO-A2-2010/028332
- US-A1- 2008 249 398
- US-A1- 2010 012 703
- US-A1- 2012 241 498
- US-A1- 2013 153 641

## Description

### CROSS-REFERENCE TO RELATED APPLICATIONS

This application claims the benefit of and priority to U.S. Provisional Patent Application No. 61/992,530, filed May 13, 2014.

The present application is a Continuation-in-Part Application claiming the benefit of and priority to U.S. Patent Application Ser. No. 14/163,386, filed on January 24, 2014, which is a Continuation-in-Part Application claiming the benefit of and priority to U.S. Patent Application Ser. No. 13/955,341, filed on July 31, 2013, which is a Continuation-in-Part Application claiming the benefit of and priority to U.S. Patent Application Ser. No. 13/419,565, filed on March 14, 2012 (U.S. Patent 8,561,873), which is a Continuation Application claiming the benefit of and priority to U.S. Patent Application Ser. No. 12/579,605, filed on October 15, 2009 (U.S. Patent 8,157,151).

U.S. Patent Application Ser. No. 13/955,341 also claims the benefit of and priority to U.S. Provisional Patent Application No. 61/696,906, filed September 5, 2012.

### BACKGROUND

### 1. Technical Field

The present disclosure relates to a surgical stapling apparatus and, more particularly, to a surgical stapling apparatus including a detachable surgical buttress for an anvil and a staple cartridge.

### 2. Background of Related Art

Surgical stapling instruments that are used to sequentially or simultaneously apply one or more rows of fasteners to join segments of body tissues are well known in the art. The fasteners are typically in the form of surgical staples but two part polymeric fasteners can also be utilized. Such devices generally include a pair of jaws to clamp therebetween the body tissues to be joined. Typically, one of the jaw members includes a staple cartridge which accommodates a plurality of staples arranged in at least two lateral rows while the other jaw member has an anvil that defines a surface for forming the staple legs as the staples are driven from the staple cartridge.

When the stapling instrument is actuated, longitudinally translating cams contact staple drive members in one of the jaws which in turn acts upon staple pushers to sequentially or simultaneously eject the staples from the staple cartridge. A blade can travel between the staple rows to longitudinally cut and/or open the stapled tissue between the rows of staples. Such instruments are disclosed, for example, in U.S. Pat. No. 3,079,606 and U.S. Pat. No. 3, 490,675.

When stapling relatively thin or fragile tissues, it is important to effectively seal the staple line against air or fluid leakage. Additionally, it is often necessary to reinforce the staple line against the tissue to prevent tears in the tissue or pulling of the staples through the tissue. One method of preventing tears or pull through involves the placement of a biocompatible fabric reinforcing material, or a "buttress," between the staple and the underlying tissue. In this method, a layer of buttress material is placed against the tissue and the tissue is stapled in conventional manner. In more recent methods, the layer of buttress is positioned on the stapling instrument itself prior to stapling the tissue. Some surgical staplers utilize fasteners or clips to temporarily connect buttress material to each of the jaws of the staplers, i.e., one disposed on the staple cartridge assembly and the other on the anvil assembly.

WO 2008/109125 discloses a surgical stapling apparatus that includes a cartridge assembly, an anvil assembly, and a surgical buttress releasebly secured by an anchor. The anchor releases the surgical buttress during firing of the stapling apparatus. EP 2305135 A1 discloses a buttress having etched wells which may serve as a reservoir for bioactive agents such as brachytherapy seeds. A polymeric layer is also provided over the buttress which may control the release of the bioactive agent from the buttress. WO 2010/028332 A2 discloses a surgical buttress comprising carrier sheet-like elements. Radioactive materials, such as brachytherapy seeds, are attached to the carrier elements.

It is a desire of the present application to provide surgical stapling apparatus with a surgical buttress secured thereto in a manner that minimizes shifting of the surgical buttress, and tearing or other damage to the surgical buttress during assembly. It would also be desirable to provide a single profile buttress that can be used on a surgical staple cartridge assembly and/or a surgical anvil cartridge assembly, as well as cartridge and anvil assemblies of different sizes. Accordingly, it is an object of this disclosure to meet the aforementioned desires.

### SUMMARY

The present disclosure relates to a surgical stapling apparatus including a surgical buttress.

According to an aspect of the present disclosure, a surgical stapling apparatus is provided and includes a cartridge assembly defining a first tissue contacting surface, the cartridge assembly housing a plurality of surgical fasteners therein, the cartridge assembly defining at least one distal attachment point and at least one proximal attachment point; an anvil assembly defining a second tissue contacting surface, the anvil assembly movably secured in relation to cartridge assembly, the anvil assembly defining at least one distal attachment point and at least one proximal attachment point, wherein the at least one proximal attachment point of the anvil assembly is offset an axial distance from the at least one proximal attachment point of the cartridge assembly; and a surgical buttress releasably secured to each of the first tissue contacting surface and the second tissue contacting surface, the surgical buttress including a body portion configured to substantially overlie at least one of the first and second tissue contacting surfaces of either the first length and second length cartridge assembly and anvil assembly.

Each surgical buttress defines a distal attachment feature for registration with the distal attachment point of the cartridge assembly and the anvil assembly; and a first proximal attachment feature and a second proximal attachment feature offset an axial distance from the first proximal attachment feature. The first proximal attachment feature registers with the proximal attachment point of the cartridge assembly; and the second proximal attachment feature registers with the proximal attachment point of the anvil assembly.

The surgical buttress may be disposed against the tissue contact surface of the cartridge assembly and the surgical buttress may be disposed against the tissue contact surface of the anvil assembly have substantially the same length.

Each surgical buttress may have the same configuration. Each surgical buttress may be fabricated from a biocompatible and bioabsorbable material.

The surgical stapling apparatus may further include sutures retaining surgical buttresses against the tissue contacting surface of the cartridge assembly and the anvil assembly.

The surgical stapling apparatus may further include a suture retaining a distal end portion of the surgical buttress against a respective one of the cartridge assembly and the anvil assembly, wherein the suture maintains the distal attachment feature of the surgical buttress in registration with the distal attachment point of the respective one of the cartridge assembly and the anvil assembly.

The surgical stapling apparatus may further include a suture retaining a proximal end portion of the surgical buttress against a respective one of the cartridge assembly and the anvil assembly, wherein a suture maintains the first proximal attachment feature of the surgical buttress in registration with the proximal attachment point of the cartridge assembly and the anvil assembly, and wherein a suture maintains the second proximal attachment feature of the surgical buttress in registration with the proximal attachment point of the anvil assembly.

According to another aspect of the present disclosure, a surgical stapling apparatus is provided and includes a cartridge assembly defining a first tissue contacting surface, the cartridge assembly housing a plurality of surgical fasteners therein, the cartridge assembly being either a first length or a second length longer than the first length; an anvil assembly defining a second tissue contacting surface, the anvil assembly movably secured in relation to cartridge assembly, the anvil assembly being either the first length or the second length each corresponding to the length of the cartridge assembly; and a surgical buttress releasably secured to at least one of the first tissue contacting surface and the second tissue contacting surface, the surgical buttress including a head portion, a neck portion, and a body portion. The head portion is connected to a distal end of the body portion by the neck portion. The surgical buttress is configured to substantially overlie at least one of the first and second tissue contacting surfaces of either the first length and second length cartridge assembly and anvil assembly.

The body portion of the surgical buttress may define a recess formed in a proximal edge thereof, the recess longitudinally bisecting the proximal edge. The recess may be a notch having a v-shape profile.

The body portion of the surgical buttress may define at least one pair of opposing proximal recesses, and each of the at least one pair of opposing proximal recesses may be formed on an opposing lateral side of the body portion near a proximal edge of the surgical buttress. The at least one pair of opposing proximal recesses may be a notch having a v-shape profile.

The body portion of the surgical buttress may further define a pair of opposing distal recesses, and each of the pair of opposing distal recesses may be formed on an opposing lateral side of the body portion near a distal edge of the body portion.

The pair of opposing distal recesses may be longitudinally tapered. A distal edge of the body portion of the surgical buttress may be arcuate. A distal edge of the body portion of the surgical buttress may have a transverse width dimension that is less than that of the rest of the body portion. The head portion may have a substantially rectangular shape.

Each surgical buttress may be fabricated from a biocompatible and bioabsorbable material.

According to a further aspect of the present disclosure, a surgical buttress for use in a surgical stapling apparatus is provided. The surgical buttress includes a body portion; a neck portion; and a head portion connected to a distal end of the body portion by the neck portion, wherein the body portion defines at least one pair of opposing proximal recesses, each of the at least one pair of opposing proximal recesses is formed on an opposing lateral side of the body portion near a proximal edge of the body portion.

Each of the at least one pair of opposing proximal recesses may be a notch having a v-shape profile. The body portion may further define a pair of opposing distal recesses, each of the pair of opposing distal recesses may be formed on an opposing lateral side of the body portion near a distal edge of the body portion.

The body portion of the surgical buttress may further define a proximal edge recess formed in a proximal edge thereof, the proximal edge recess longitudinally bisecting the proximal edge. A distal edge of the body portion of the surgical buttress may be arcuate. A distal edge of the body portion of the surgical buttress may have a transverse width dimension less than that of the rest of the body portion. The head portion may have a substantially rectangular shape.

The surgical buttress may be fabricated from a biocompatible and bioabsorbable material.

According to still another aspect of the present disclosure, a surgical buttress is provided for use with a surgical stapling apparatus having a cartridge assembly of any number of lengths and an anvil assembly of any number of lengths corresponding to the lengths of the cartridge assembly, wherein each of the cartridge assembly and anvil assembly defines respective juxtaposed tissue contacting surfaces, and wherein the cartridge assembly includes a plurality of staples stored in staple slots thereof for formation against staple formation pockets of the anvil assembly. The surgical buttress includes a body portion configured and dimensioned to overlie all of the staple slots of the cartridge assembly for any length cartridge assembly, and/or overlie all of the staple formation pockets of the anvil assembly for any length anvil assembly. The surgical buttress further includes a neck portion extending from the body portion; and a head portion connected to the neck portion and opposite a distal end of the body portion, wherein the body portion defines at least one pair of opposing proximal recesses, each of the at least one pair of opposing proximal recesses is formed on an opposing lateral side of the body portion near a proximal edge of the body portion.

According to yet another aspect of the present disclosure a surgical buttress for use in a surgical stapling apparatus is provided and includes an elongate rectangular body portion defining a width; a neck portion integrally formed with and extending from a distal end of the body portion, the neck portion defining a width; a head portion integrally formed with and connected to a distal end of the neck portion, the head portion defining a width; and a tail portion integrally formed with and extending from a proximal end of the body portion, the tail portion defining a width. The width of the tail portion is less than the width of the body portion, and the surgical buttress is formed from a material having filaments.

In certain embodiments, the width of the neck portion is less than the width of the body portion, or the width of the neck portion is less than the width of the tail portion, or the width of the neck portion is more than one-half the width of the body portion.

A length of the head portion may be greater than a length of the tail portion. A length of the head portion may be greater than a length of the neck portion.

The tail portion can define at least one pair of opposing proximal recesses, each of the at least one pair of opposing proximal recesses is formed on an opposing lateral side of the tail portion.

The body portion can further define a pair of opposing distal recesses, each of the pair of opposing distal recesses is formed on an opposing lateral side of the body portion near a distal edge of the body portion.

The tail portion of the surgical buttress can define a proximal edge recess formed in a proximal edge thereof, wherein the proximal edge recess longitudinally bisects the proximal edge.

The surgical buttress is desirably fabricated from a biocompatible and bioabsorbable material.

The surgical buttress can be fabricated from a material selected from the group consisting of polyglycolic acid, glycolide trimethylene carbonate, polyglycolic acid trimethylene carbonate, and blends thereof. The surgical buttress can be formed as a non-woven material.

According to a further aspect of the present disclosure, a surgical buttress is provided for use with a surgical stapling apparatus having a cartridge assembly of any number of lengths and an anvil assembly of any number of lengths corresponding to the lengths of the cartridge assembly, wherein each of the cartridge assembly and anvil assembly defines respective juxtaposed tissue contacting surfaces, and wherein the cartridge assembly includes a plurality of staples stored in staple slots thereof for formation against staple formation pockets of the anvil assembly. The surgical buttress includes an elongate rectangular body portion defining a length and a width; a neck portion integrally formed with and extending from a distal end of the body portion, the neck portion defining a length and a width; a head portion integrally formed with and connected to a distal end of the neck portion, the head portion defining a length and a width; and a tail portion integrally formed with and extending from a proximal end of the body portion, the tail portion defining a length and a width. The width of the tail portion is less than the width of the body portion. The body portion and the tail portion are configured and dimensioned to overlie all of the staple slots of the cartridge assembly for any length cartridge assembly, and/or overlie all of the staple formation pockets of the anvil assembly for any length anvil assembly.

The width of the neck portion may be less than the width of the body portion. The width of the neck portion may be less than the width of the tail portion. The width of the neck portion may be more than one-half the width of the body portion.

The length of the head portion may be greater than the length of the tail portion. The length of the head portion may be greater than the length of the neck portion.

The tail portion may define at least one pair of opposing proximal recesses. Each of the at least one pair of opposing proximal recesses may be formed on an opposing lateral side of the tail portion.

The body portion may further define a pair of opposing distal recesses. Each of the pair of opposing distal recesses may be formed on an opposing lateral side of the body portion near a distal edge of the body portion.

The tail portion of the surgical buttress may define a proximal edge recess formed in a proximal edge thereof. The proximal edge recess may longitudinally bisect the proximal edge.

The surgical buttress may be fabricated from a biocompatible and bioabsorbable material. The surgical buttress may be fabricated from a material selected from the group consisting of polyglycolic acid, glycolide trimethylene carbonate, polyglycolic acid trimethylene carbonate, and blends thereof. The surgical buttress can be made as a non-woven material.

According to another aspect of the present disclosure, a surgical buttress for use in a surgical stapling apparatus is provided. The surgical buttress includes an elongate rectangular body portion defining a width; a nose portion integrally formed with and extending from a distal end of the body portion, the nose portion defining a width that is less than the width of the body portion; a neck portion integrally formed with and extending from a distal end of the body portion, the neck portion defining a width; a head portion integrally formed with and connected to a distal end of the neck portion, the head portion defining a width; and a tail portion integrally formed with and extending from a proximal end of the body portion, the tail portion defining a width that is less than the width of the body portion. The surgical buttress is formed from a material having filaments.

The width of the neck portion may be less than the width of the nose portion. The width of the neck portion may be less than the width of the tail portion. The width of the neck portion may be more than one-half the width of the body portion.

The tail portion may define at least one pair of opposing proximal recesses, each of the at least one pair of opposing proximal recesses is formed in an opposing lateral side of the tail portion.

The at least one pair of opposing proximal recesses may include a first pair of opposing proximal recesses; and a second pair of opposing proximal recesses, wherein the first pair of opposing proximal recesses is disposed proximal of the second pair of opposing proximal recesses.

Each of the second pair of opposing proximal recesses may extend towards a longitudinal axis of the buttress a greater amount than each of the first pair of opposing proximal recesses.

A side edge of the tail portion, disposed between the first pair of opposing proximal recesses and the pair of opposing proximal recesses, may taper towards a longitudinal axis of the buttress from a proximal end to a distal end.

Each of the first pair of opposing proximal recesses and each of the second pair of opposing proximal recesses may have a v-shaped profile. A distal edge of each of the first pair of opposing proximal recesses and each of the second pair of opposing proximal recesses may be oriented orthogonal to a longitudinal axis of the buttress.

The body portion may further define a pair of opposing distal recesses. Each of the pair of opposing distal recesses may be formed in opposing lateral sides of the nose portion.

The tail portion of the surgical buttress may define a proximal edge recess formed in a proximal edge thereof, wherein the proximal edge recess may longitudinally bisect the proximal edge.

The surgical buttress may be fabricated from a biocompatible and bioabsorbable material. The surgical buttress may be fabricated from a material selected from the group consisting of polyglycolic acid, glycolide trimethylene carbonate, polyglycolic acid trimethylene carbonate, and blends thereof. The surgical buttress may be formed as a non-woven material.

According to still another aspect of the present disclosure, a surgical buttress for use with a surgical stapling apparatus is provided. The surgical stapling apparatus includes a cartridge assembly of any number of lengths and an anvil assembly of any number of lengths corresponding to the lengths of the cartridge assembly, wherein each of the cartridge assembly and anvil assembly defines respective juxtaposed tissue contacting surfaces, and wherein the cartridge assembly includes a plurality of staples stored in staple slots thereof for formation against staple formation pockets of the anvil assembly.

The surgical buttress includes an elongate rectangular body portion defining a width; a nose portion integrally formed with and extending from a distal end of the body portion, the nose portion defining a width that is less than the width of the body portion; a neck portion integrally formed with and extending from a distal end of the body portion, the neck portion defining a width; a head portion integrally formed with and connected to a distal end of the neck portion, the head portion defining a width; and a tail portion integrally formed with and extending from a proximal end of the body portion, the tail portion defining a width that is less than the width of the body portion.

The body portion and the tail portion are configured and dimensioned to at least one of overlie all of the staple slots of the cartridge assembly for any length cartridge assembly, and overlie all of the staple formation pockets of the anvil assembly for any length anvil assembly.

The width of the neck portion may be less than the width of the nose portion.

The tail portion may define at least one pair of opposing proximal recesses. Each of the at least one pair of opposing proximal recesses may be formed in an opposing lateral side of the tail portion.

The at least one pair of opposing proximal recesses may include a first pair of opposing proximal recesses; and a second pair of opposing proximal recesses, wherein the first pair of opposing proximal recesses is disposed proximal of the second pair of opposing proximal recesses.

Each of the second pair of opposing proximal recesses may extend towards a longitudinal axis of the buttress a greater amount than each of the first pair of opposing proximal recesses.

A side edge of the tail portion, disposed between the first pair of opposing proximal recesses and the pair of opposing proximal recesses, may taper towards a longitudinal axis of the buttress from a proximal end to a distal end.

Each of the first pair of opposing proximal recesses and each of the second pair of opposing proximal recesses may have a v-shaped profile. A distal edge of each of the first pair of opposing proximal recesses and each of the second pair of opposing proximal recesses may be oriented orthogonal to a longitudinal axis of the buttress.

The body portion may further define a pair of opposing distal recesses. Each of the pair of opposing distal recesses may be formed in opposing lateral sides of the nose portion.

The tail portion of the surgical buttress may define a proximal edge recess formed in a proximal edge thereof, wherein the proximal edge recess longitudinally bisects the proximal edge.

The surgical buttress may be formed from a non-woven material.

According to another aspect of the present disclosure, a surgical buttress for use in a surgical stapling apparatus is provided. The surgical buttress includes an elongate rectangular body portion defining a width and being fabricated from at least one of a biocompatible or bioabsorbable material. The surgical buttress is formed with at least one marking provided at least one of in or on the body portion.

The at least one marking may be formed as a notch.

The at least one marking may include biocompatible and bioabsorbable materials.

The at least one marking may include a biocompatible ink.

The at least one marking may be sewn into the surgical buttress.

The body portion may define at least one pair of notch markings on opposing lateral sides of the surgical buttress.

The body portion may include a plurality of markings. The plurality of markings may be formed by physical notches and ink. The plurality of markings may vary in length. The plurality of markings may be evenly spaced along a length of the surgical buttress.

The surgical buttress may further include a radioactive material embedded within the body portion. The radioactive material may be at least one brachytherapy seed.

The body portion may include a plurality of pores or pockets. The at least one pore may contain a brachytherapy seed.

According to still another aspect of the present disclosure, a surgical buttress for use in a surgical stapling apparatus is provided. The surgical buttress includes a body portion; and radioactive material provided at least one of in or on the body portion.

The body portion may include biocompatible and bioabsorbable materials.

The radioactive material may be at least one brachytherapy seed.

The body portion may include a plurality of pores. At least one pore may contain a brachytherapy seed.

The surgical buttress may be formed with at least one marking provided at least one of in or on the body portion.

According to still another aspect of the present disclosure, a method of embedding radioactive material into a surgical buttress is provided. The method includes providing a surgical buttress; providing a template capable of holding the radioactive material; inserting the radioactive material into the template; mating the template to the surgical buttress; and embedding the radioactive material into the surgical buttress.

The method may further include providing a surgical buttress comprising a plurality of pores or pockets.

The method may further include providing a template that is capable of receiving at least one brachytherapy seed.

The method may further include inserting brachytherapy seeds.

The method may further include embedding the radioactive material into at least 10% of a surface area of the buttress.

The method may further include embedding the radioactive material into a distal end of the surgical buttress.

The method may further include embedding the radioactive material into a proximal end of the surgical buttress.

The method may further include embedding the radioactive material uniformly across a surface area of the surgical buttress.

### BRIEF DESCRIPTION OF THE DRAWINGS

The present disclosure will be further described with reference to the accompanying drawings, wherein like reference numerals refer to like parts in the several views, and wherein:
FIG. 1 is a perspective view of a surgical stapling apparatus according to an embodiment of the present disclosure;
FIG. 2 is a top, exploded, perspective view of a distal end of a DLU of the surgical stapling apparatus of FIG. 1;
FIG. 3 is a top plan view of a buttress according to an embodiment of the present disclosure;

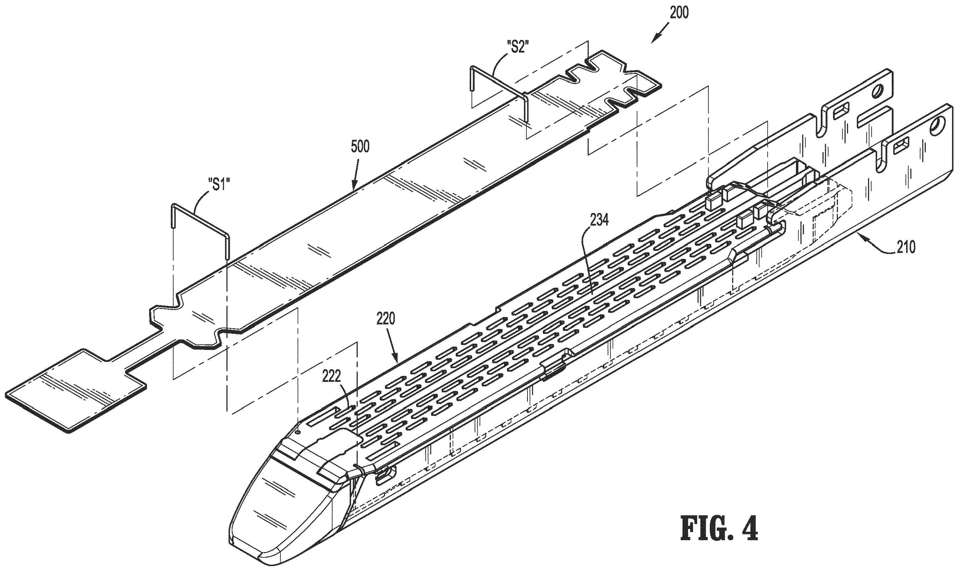

FIG. 4 is a top, perspective view of a cartridge half-section of the DLU of FIG. 2;

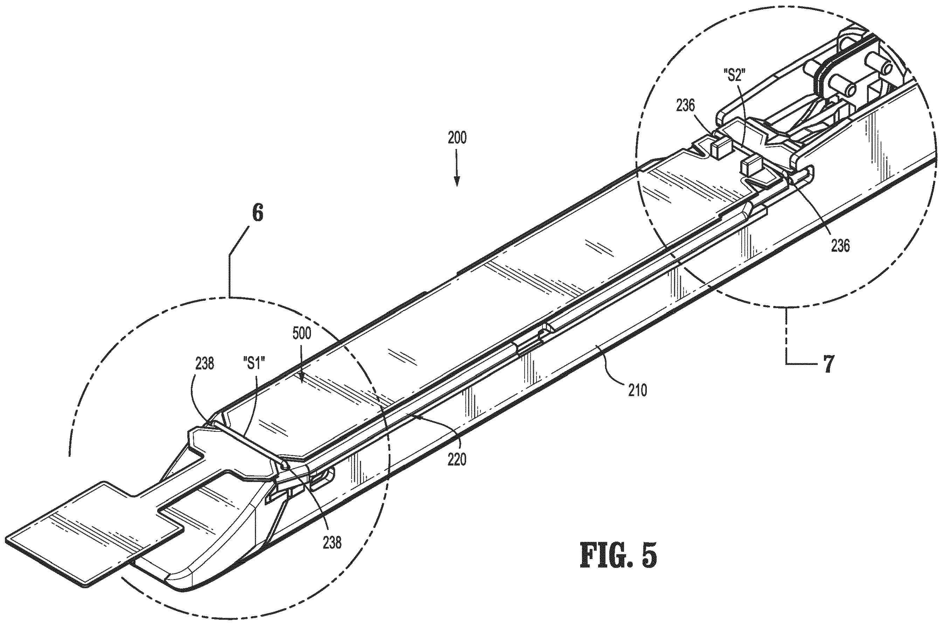

FIG. 5 is a perspective view of a cartridge assembly of the DLU of FIG. 2;

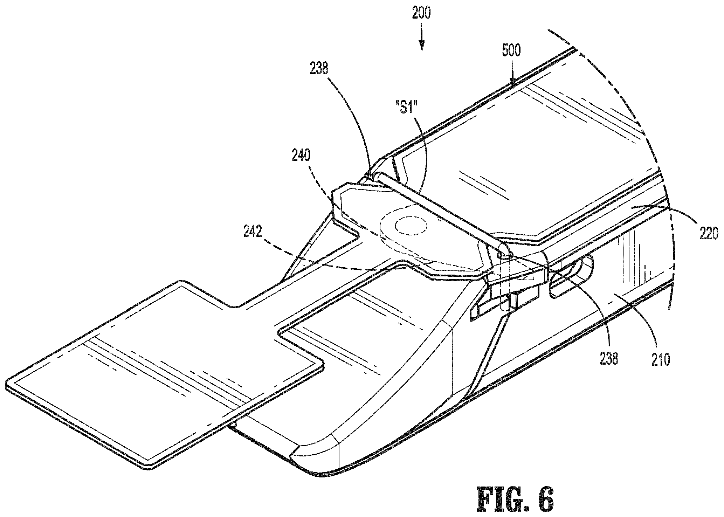

FIG. 6 is an enlarged perspective view of a distal end of the cartridge assembly of FIG. 5;

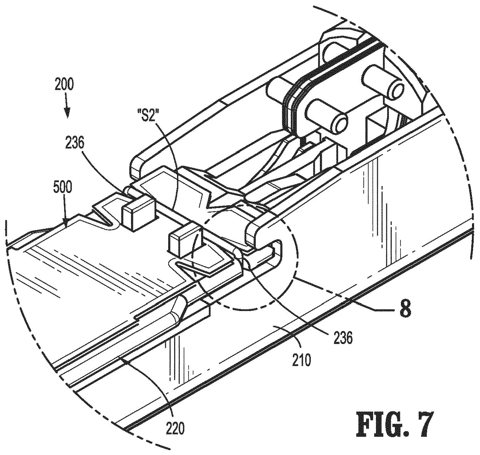

FIG. 7 is an enlarged view of the indicated area of detail of FIG. 5;

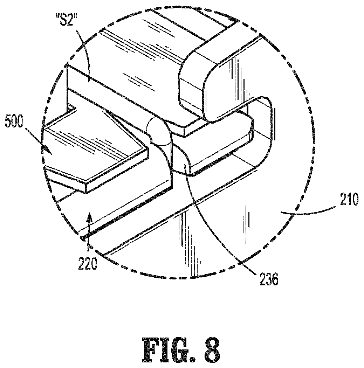

FIG. 8 is an enlarged view of the indicated area of detail of FIG. 7;
FIG. 9 is a perspective view of an anvil assembly of the DLU of FIG. 2, illustrating the buttress of FIG. 3 secured thereto;
FIG. 10 is a plan view of the cartridge assembly of the DLU of FIGS. 4-8 and the anvil assembly of the DLU of FIG. 9, illustrating the attachment of the buttress of FIG. 3 at different attachment points of a respective cartridge assembly and anvil assembly;
FIG. 11 is a top plan view of a buttress according to another embodiment of the present disclosure;
FIG. 12 is a top plan view of a buttress according to yet another embodiment of the present disclosure;
FIG. 13 is an enlarged view of the indicated area of detail of FIG. 12;
FIG. 14 is an enlarged view of the indicated area of detail of FIG. 12;
FIG. 15 is a top plan view of a buttress according to another embodiment of the present disclosure;
FIG. 16 is a top plan view of a buttress according to another embodiment of the present disclosure;
FIG. 17 is a top plan view of a buttress according to another embodiment of the present disclosure;
FIG. 18 is a top plan view of a buttress according to another embodiment of the present disclosure;
FIG. 19 is a top plan view of a buttress according to another embodiment of the present disclosure;
FIG. 20 is a cross-sectional view, as taken through 20-20 of FIG. 19;
FIG. 21 is an enlarged view of the indicated area of detail of FIG. 19;
FIG. 22 is a top plan view of a buttress according to another embodiment of the present disclosure;
FIG. 23 is a top plan view of a surgical buttress according to certain embodiments of the present disclosure;
FIG. 24 is a perspective view of a staple cartridge assembly having a surgical buttress attached thereto in accordance with embodiments of the present disclosure; and
FIG. 25 is a perspective detail view of one of the pockets of a surgical buttress according to certain embodiments of the present disclosure.

### DETAILED DESCRIPTION OF EMBODIMENTS

Embodiments of the presently disclosed staple line reinforcement for anvil and cartridge of a loading unit of a surgical stapling apparatus will now be described in detail with reference to the drawings, in which like reference numerals designate identical or corresponding elements in each of the several views. In the drawings and in the description that follows, the term "proximal," as is traditional, will refer to the end of the stapling apparatus which is closest to the operator, while the term "distal" will refer to the end of the apparatus which is farthest from the operator.

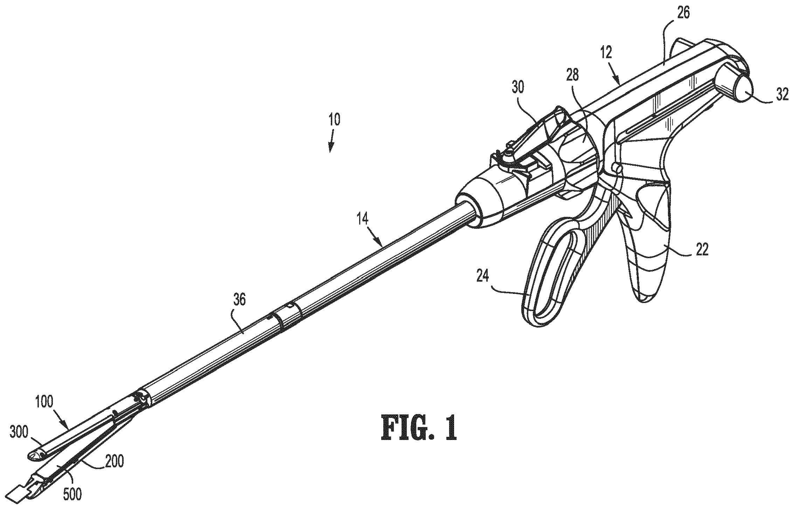

Referring now to FIG. 1, there is disclosed a linear surgical stapling apparatus, generally referred to as 10. In the interest of brevity, this disclosure will focus primarily on a buttress utilized in a loading unit 100, e.g., a single use loading unit ("SULU") or a disposable loading unit ("DLU"). For simplicity, hereinafter, SULU or DLU will be referred to as "DLU," but it should be understood to include either or both a DLU or SULU. An exemplary example of this type of surgical stapling instrument is disclosed in U.S. Patent No. 7,128,253.

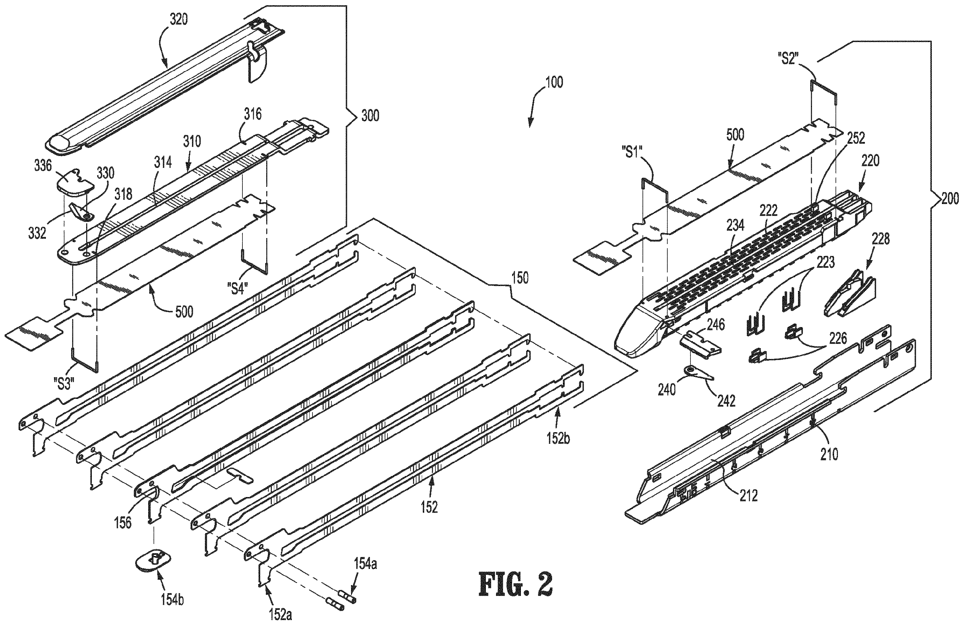

Surgical stapling apparatus 10 generally includes a handle assembly 12 and an elongate body 14 extending distally from handle assembly 12. A DLU 100 is releasably secured to the distal end of elongate body 14. DLU 100 includes a cartridge assembly 200 housing a plurality of surgical fasteners or staples 223 (see FIG. 2) and an anvil assembly 300 movably secured in relation to cartridge assembly 200. Handle assembly 12 includes a stationary handle member 22, a movable handle member 24, and a barrel portion 26. An articulation lever 30 is mounted on the forward end of barrel portion 26 adjacent rotatable member 28 to facilitate articulation of DLU 100. A pair of knobs 32 is movably positioned along barrel portion 26. Knobs 32 are advanced distally to approximate or close cartridge and/or anvil assembly 200, 300, and retracted proximally to unapproximate or open cartridge and/or anvil assembly 200, 300. Actuation of movable handle member 24 applies lines of staples 223 to tissue. In order to properly orient cartridge and anvil assembly 200, 300 relative to the tissue to be stapled, surgical stapling apparatus 10 is additionally provided with a rotatable member 28 on the forward end of barrel portion 26. Rotation of rotatable member 28 relative to handle assembly 12 rotates elongate body 14 and loading unit 100 relative to handle assembly 12 so as to properly orient cartridge assembly 200 and anvil assembly 300 relative to the tissue to be stapled.

As seen in FIG. 2, cartridge assembly 200 includes a carrier 210 defining an elongated support channel 212. Elongated support channel 212 of carrier 210 is dimensioned and configured to selectively receive a staple cartridge 220 therein. Staple cartridge 220 includes retention slots 222 formed therein for receiving a plurality of fasteners 223 and pushers 226. A plurality of spaced apart longitudinal slots extend through staple cartridge 220 to accommodate upstanding cam wedges of actuation sled 228. A central longitudinal slot 234 is formed in and extends along the length of staple cartridge 220 to facilitate passage of knife blade 156 of drive bar 150 therethrough. During operation of surgical stapler 10, actuation sled 228 translates through staple cartridge 220 to advance the cam wedges into sequential contact with pushers 226, to cause pushers 226 to translate vertically within retention slots 222 and urge staples 223 from slots 222 into staple forming cavities of anvil plate 310 of anvil assembly 300.

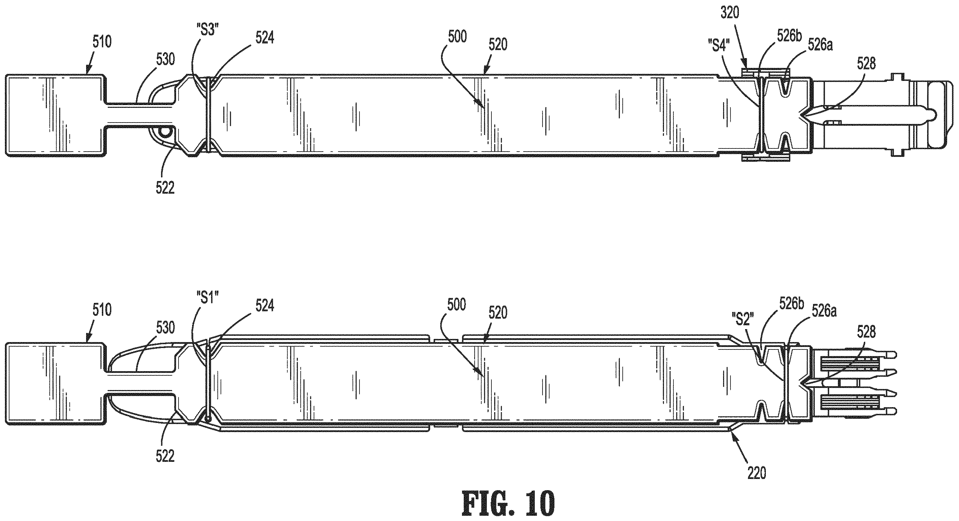

As seen in FIG. 2, cartridge assembly 200 includes a surgical cartridge buttress 500 operatively secured to an upper surface of staple cartridge 220, by sutures "S1, S2," to overlie at least some of retention slots 222 and/or at least a portion of a length of longitudinal slot 234. A first suture "S1" is threaded through each of a distal pair of recesses or attachment points 238 and around/over distal portion of cartridge buttress 500 and, and a second suture "S2" is threaded through each of a proximal pair of recesses or attachment points 236 and around/over proximal portion of cartridge buttress 500. A first end of each suture "S1, S2" may be anchored or fixed in a respective one recesses of the proximal and distal pair of recesses or attachment points 236, 238 while a second end of each suture "S1, S2" passes transversely across respective distal and proximal portions of cartridge buttress 500 and is anchored or fixed in a respective other recess of the proximal and distal pair of recesses or attachment points 236, 238. As seen in FIG. 10, cartridge assembly 200 defines an axial distance "D1" between the distal pair of recesses or attachment points 238 and the proximal pair of recesses or attachment points 236.

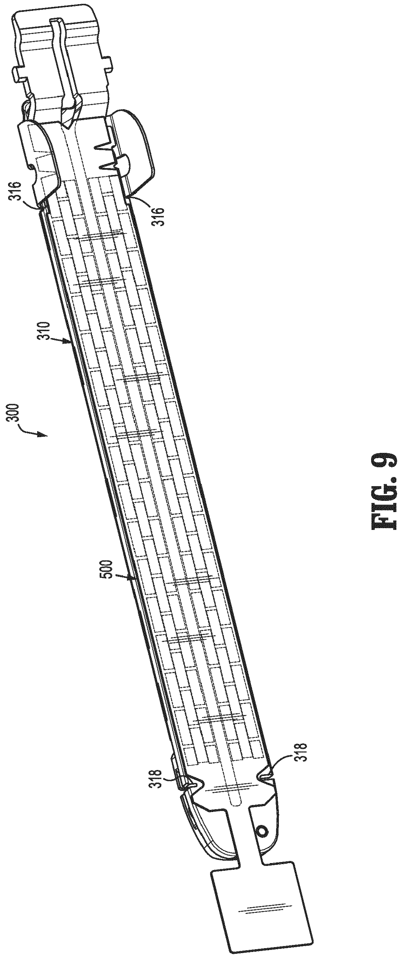

With reference still to FIG. 2, anvil assembly 300 includes an anvil plate 310 having a plurality of staple deforming pockets/cavities 310a (see FIG. 9) and a cover plate 320 secured to a top surface of anvil plate 310. Anvil assembly 300 further includes a knife blade 330 operatively interposed within the cavity defined between anvil plate 310 and cover plate 320.

Anvil plate 310 defines a proximal pair of recesses or attachment points 316 formed near a proximal end of anvil plate 310 and disposed, one each, on opposed sides of longitudinal slot 314. Anvil plate 310 defines a distal pair of recesses or attachment points 318 formed near a distal end of anvil plate 310 and disposed, one each, on opposed sides of longitudinal slot 314. At least one recess of each of the proximal pair of recesses or attachment points 316 and the distal pair of recesses or attachment points 318 is in the form of a slot or notch having a constricting profile so as to frictionally engage and/or pinch a suture "S". Anvil assembly 300 further includes a surgical anvil buttress 500 operatively secured to a lower surface of anvil plate 310, by sutures "S3, S4," to overlie at least some of anvil pockets 310a and/or at least a portion of a length of longitudinal slot 314.

With reference still to FIG. 2, anvil buttress 500 is secured to a lower surface of anvil plate 310, by anchors "S3, S4", to overlie at least some of the anvil pockets and/or at least a portion of a length of longitudinal slot 314. In particular, an anchor "S3" is threaded across a distal portion of the anvil buttress 500 and each of the corresponding distal pair of recesses or attachment points 318, and an anchor "S4" is threaded across a proximal portion of anvil buttress 500 and each of the corresponding proximal pair of recesses or attachment points 316. As seen in FIG. 10, anvil assembly 300 defines an axial distance "D2" between the distal pair of recesses or attachment points 318 and the proximal pair of recesses or attachment points 316.

Reference may be made to U.S. Patent Application Serial No. 12/342,400, filed on December 23, 2008 (U.S. Patent No. 8,011,555), for a detailed discussion of the construction and operation of surgical stapling apparatus 10, cartridge assembly 200 and/or anvil assembly 300.

It is contemplated that the cartridge buttress and/or anvil buttress can be attached by other means. For example, in any of the embodiments disclosed herein, the buttresses can be attached using adhesives, welding, and/or attachment features incorporated in the buttress material.

Buttress 500 for each of cartridge assembly 200 and anvil assembly 300 is provided to reinforce and seal staple lines applied to tissue by surgical stapling apparatus 10. Cartridge assembly 200 and anvil assembly 300 are particularly configured to allow surgical buttresses 500 to be localized on inwardly facing surfaces of cartridge assembly 200 and anvil assembly 300 in order to facilitate passage of surgical stapling apparatus 10 into the body of a patient without risk of tearing or wrinkling of the respective buttresses as surgical stapling apparatus 10 is inserted into and manipulated within the body of a patient. The material from which the buttress 500 is formed may be bioabsorbable or non-bioabsorbable. It should be understood that any combination of natural, synthetic, bioabsorbable and non-bioabsorbable materials may be used to form the buttress material. The buttress material may be porous or non-porous, or a combination of porous and non-porous layers. The non-porous buttress material may be utilized to retard or prevent tissue ingrowth from surrounding tissues thereby acting as an adhesion barrier and preventing the formation of unwanted scar tissue.

Additional exemplary materials for surgical buttresses 500 for use with the surgical stapling devices disclosed herein are set forth in commonly assigned U.S. Patent Nos. 5,542,594; 5,908,427; 5,964,774; and 6,045,560, and commonly assigned U.S. Application Publication Nos. 2006/0085034, filed on April 20, 2006; and 2006/0135992, filed on June 22, 2006.

In an embodiment, surgical buttresses 500 may be fabricated from a suitable biocompatible and bioabsorbable material. Surgical buttresses 500 may also be fabricated from a non-absorbent material which does not retain fluid, for example, surgical buttresses 500 may be fabricated from "BIOSYN™" (a synthetic polyester, commercially available from Tyco Healthcare Group, LP d/b/a COVIDIEN, North Haven, CT), which is made from "GLYCOMER 631" (a block copolymer) which is a synthetic polyester composed of glycolide, dioxanone and trimethylene carbonate.

One block of the resulting copolymer contains randomly combined units derived from p-dioxanone (1,4-dioxan-2-one) and trimethylene carbonate (1,3-dioxan-2-one). A second block of the copolymer contains randomly combined units derived from glycolide and p-dioxanone. The resulting polyester is an ABA triblock terpolymer possessing about 60% glycolide, about 14% dioxanone, and about 26% trimethylene carbonate.

Anvil buttress and/or cartridge buttress 500 may be pre-loaded (i.e., from the manufacturer) onto anvil assembly 300 or cartridge assembly 200. Additional or replacement buttresses 500 for anvil assembly 300 and/or cartridge assembly 200 may be secured to either anvil assembly 300 or cartridge assembly 200 as needed or desired.

In operation, with DLU 100 coupled to a distal end of elongated body 14 of surgical stapling apparatus 10, and with anvil and cartridge buttresses 500 pre-loaded onto anvil assembly 300 and cartridge assembly 200, respectively, surgical stapling apparatus 10 is used in accordance with methods known by those skilled in the art. Once anvil assembly 300 and cartridge assembly 200 are clamped onto tissue, surgical stapling apparatus 10 is fired. In firing surgical stapling apparatus 10, drive bar 150 is advanced from a proximal-most position to a distal-most position of DLU 100. In so doing, knife blade 156 of drive bar 150 enters notch 528 of buttress 500 thereby facilitating the dividing of buttress 500 and reducing any incidents of pushing or bunching-up of buttress 500 by blade 156. As drive bar 150 begins to travel distally, knife blade 156 substantially simultaneously cuts through a central section of the proximal anchors "S2, S4" of anvil assembly 300 and cartridge assembly 200, thereby respectively freeing the proximal ends of anvil and cartridge buttresses 500 therefrom. As knife blade 156 is moved distally, knife blade 156 slices or cuts longitudinally through both anvil buttress 500 and cartridge buttress 500, thereby dividing the buttresses 500 substantially in half.

Additionally, as drive bar 150 approaches the distal-most position, drive bar 150 and/or knife blade 156 engage a suture cutting assembly or suture release assembly, as described in U.S. Patent Application Serial No. 12/342,400, filed on December 23, 2008, to thereby sever or release distal sutures "S1 or S3" and thus release a distal end of buttress 500.

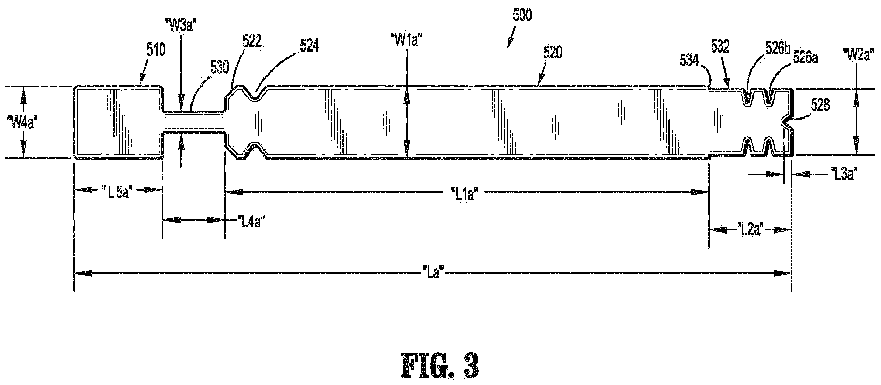

With reference to FIG. 3, an embodiment of a surgical buttress 500 having a uniform profile in accordance with the present disclosure is illustrated. Buttress 500 includes a head portion 510, a body portion 520, a neck portion 530 interconnecting head portion 510 and body portion 520, and a tail portion 532 extending proximally from body portion 520.

Buttress 500 is configured to be detachably secured to any sized anvil assembly 300 and/or cartridge assembly 200, as described above. Body portion 520 of buttress 500 defines a pair of opposing distal recesses 524 on transverse edges near a distal location 522 thereof. The pair of opposing distal recesses 524 may be utilized to secure body portion 520 to a distal end of anvil assembly 300 and/or cartridge assembly 200, either through a use of suture "S1 or S3" or any other type of fastener, e.g., staple. Distal portion 522 of body portion 520 has a reduced transverse cross-sectional dimension, e.g., angled, arcuate, so as to be suitable for various types of anvil and cartridge assemblies having different shapes.

Tail portion 532 of buttress 500 defines two pairs of opposing proximal recesses 526a, 526b formed therein. Each of the pair of proximal recesses 526a, 526b is disposed on a transverse side of tail portion 532 near the proximal edge thereof. Such proximal pair of recesses 526a, 526b serve to detachably secure tail portion 532 of buttress 500 to a proximal end of anvil assembly 300 and/or cartridge assembly 200. In order to accommodate various types of profiles, tail portion 532 of buttress 500 preferably has been provided with two pairs of opposing recesses, a first proximal pair of recesses 526a, and a second proximal pair of recesses 526b (located distal of the first proximal pair of recesses 526a). Each of the proximal pair of recesses 526a, 526b has a substantially v-shape profile.

In particular, when buttress 500 is to be used with a relatively longer anvil assembly 300 and/or cartridge assembly 200, then a suture "S2 or S4" is extended across tail portion 532 of buttress 500, passed through the proximal-most pair of recesses 526a of buttress 500, and secured to respective recesses 316 of anvil assembly 300 and/or recesses 236 of cartridge assembly 200. Moreover, when buttress 500 is to be used with a relatively shorter anvil assembly 300 and/or cartridge assembly 200, then a suture "S2 or S4" is extended across tail portion 532 of buttress 500, passed through the distal pair 526b of the proximal pair of recesses of buttress 500, and secured to respective recess 316 of anvil assembly 300 and/or recesses 236 of cartridge assembly 200.

According to another aspect of the present disclosure, a single profile or configuration buttress 500 may be used in connection with cartridge assembly 200 and/or anvil assembly 300. For example, the buttresses 500 that are used in connection with cartridge assembly 200 and anvil assembly 300 each may have the same overall length, width, thickness, perimetrical profile and material of construction.

In particular, as seen in FIG. 10, when buttress 500 is used in connection with cartridge assembly 200, a suture "S1" may extend transversely across a distal end portion of cartridge assembly 200 and captures or is otherwise secured to distal pair of recesses or attachment points 238 of cartridge assembly 200, wherein suture "S1" is in registration with distal recesses 524 of secure a distal end of body portion 520 of buttress 500. Additionally, when buttress 500 is used in connection with cartridge assembly 200, a suture "S2" may extend transversely across a proximal end portion of cartridge assembly 200 and captures or is otherwise secured to each of a proximal pair of recesses or attachment points 236 of cartridge assembly 200, wherein suture "S2" is in registration with the proximal-most pair 526a of the proximal pair of recesses of tail portion 532 of buttress 500.

With continued reference to FIG. 10, when buttress 500 is used in connection with anvil assembly 300, a suture "S3" may extend transversely across a distal end portion of anvil assembly 300 and captures or is otherwise secured to distal pair of recesses or attachment points 318 of anvil assembly 300, wherein suture "S3" is in registration with distal recesses 524 of secure a distal end of body portion 520 of buttress 500. Additionally, when buttress 500 is used in connection with anvil assembly 300, a suture "S4" may extend transversely across a proximal end portion of anvil assembly 300 and captures or is otherwise secured to each of a proximal pair of recesses or attachment points 316 of anvil assembly 300, wherein suture "S4" is in registration with the distal pair 526b of the proximal pair of recesses of tail portion 532 of buttress 500.

Neck portion 530 connects head portion 510 to a distal end of body portion 520. Generally, head portion 510 is in a substantially rectangular shape which is used as a tab to facilitate placement of buttress 500 in position on anvil assembly 300 and/or cartridge assembly 200. Following placement of buttress 500 on anvil assembly 300 and/or cartridge assembly 200, head portion 510 and neck portion 530 may be torn or otherwise cut away from body portion 520.

Tail portion 532 of buttress 500 includes a notch 528 at a proximal edge thereof. Notch 528 is substantially centered with respect to the longitudinal axis. Notch 528 which has a triangular or V-shape configuration may be utilized to provide a lead-in for the knife during a cutting of buttress 500. Notch 528 has a length "L3a", as seen in FIG. 3.

As seen in FIG. 3, buttress 500 has an overall length "La". Body portion 520 of buttress 500 has a length "L1a", and tail portion 532 has a length "L2a". Body portion 520 has a width "W1a" and tail portion 532 has a width "W2a" which is less that width "W1a" of body portion 520, wherein a shoulder 534 is defined between a side edge of body portion 520 and a side edge of tail portion 532. It is contemplated that a shoulder 534 is provided or defined along each opposed side edge of buttress 500.

With continued reference to FIG. 3, neck portion 530 of buttress 500 has a length "L4a", and head portion 510 has a length "L5a". Neck portion 530 of buttress 500 has a width "W3a" which is less that width "W1a" of body portion 520 and less than width "W2a" of tail portion 532. Also, head portion 510 has a width "W4a" which is substantially equal to width "W1a" of body portion 520.

The uniform profile of buttress 500 which simultaneously satisfies the requirements of one or more different assemblies offers advantages of simplifying the assembly process, minimizing the total number of unique components, and reducing assembly costs associated therewith.

Moreover, buttress 500 may be used on or in connection with cartridge assembly 200 and/or anvil assembly 300. In this manner, a single profile buttress 500 is produced and used for cartridge assembly 200 and/or anvil assembly 300. As such, the manufacturing and storage costs for buttresses 500 may be reduced.

As mentioned above, DLU 100 includes an anvil surgical buttress 500 and a cartridge surgical buttress 500 pre-loaded onto anvil assembly 300 and cartridge assembly 200. An exemplary method of loading anvil assembly 300 and/or cartridge assembly 200 with a buttress 500 will now be described.

During the manufacturing and/or assembly process of DLU 100, loading of anvil assembly 300 and/or cartridge assembly 200 with buttress 500 includes the step of placing a buttress 500 atop a tissue contacting surface of anvil assembly 300 and/or cartridge assembly 200 such that notch 528 of buttress is disposed near a proximal end of anvil assembly 300 and/or cartridge assembly 200 and head portion 510 of buttress 500 extends from a distal end of anvil assembly 300 and/or cartridge assembly 200. With buttress 500 so positioned against anvil assembly 300 and/or cartridge assembly 200, proximal sutures "S2" and/or "S4" are pulled down. Tension is then applied to buttress 500, in a distal direction, by pulling on head portion 510. Distal sutures "S1" and/or "S3" are then pulled down. At this time, all the sutures "S1-S4" are cinched in the respective attachment points 316, 318, 236, 238 of anvil assembly 300 and/or cartridge assembly 200. Following cinching of sutures "S1-S4," head portion 510 of buttress 500 may be released. Next, DLU 100 may be removed from a nesting and head portion 510 and neck portion 530 of buttress 500 may be removed or severed from body portion 520.

While the above-described embodiments surgical staplers incorporating the use of movable knife blades to sever and release surgical buttresses from the tissue contacting surfaces of the anvil assembly and the cartridge assembly have been shown and described in relation to endoscopic surgical staplers, it is envisioned and within the scope of the present disclosure that any of the embodiments disclosed herein may be incorporated into any type of surgical stapler, including and not limited to open surgical staplers, such as, for example, linear surgical staplers, circular staplers, and transverse surgical staplers.

Moreover, while only distal and proximal sutures have been shown and described as securing buttress 500 to anvil assembly 300 and/or cartridge assembly 300, it is contemplated that, in any of the embodiments disclosed herein, any number of transverse sutures may be provided along a length of the anvil assembly and/or cartridge assembly to aid with the securement of buttress 500 along a length thereof.

Any of the surgical buttresses disclosed herein may be comprised of the GLYCOMER 631 a block copolymer, or other polymers discussed above, as a film, non-woven, mesh or other type of material, and may also be made as a film, non-woven, mesh or other type of material, from poly-L-lactide (PLL), or Polycaprolactam (Nylon-6), or polyglycolic acid (PGA) each of which are homopolymers, or from glycolide trimethylene carbonate (Gly-TMC), which is a copolymer, PLL and Gly-TMC both being bio-degradable polyesters polymerized through a ring opening reaction. The non-woven material can be made utilizing a melt blown or spun bond process, or other known process. Non-woven materials and polymers are disclosed in U.S. Patent Application No. 13/293,215, entitled Hydrophilic Medical Devices, filed November 10, 2011 (U.S. Patent Publication No. 2013-0123816). In certain embodiments, non-woven, felted, or other relatively supple materials having filaments are preferred.

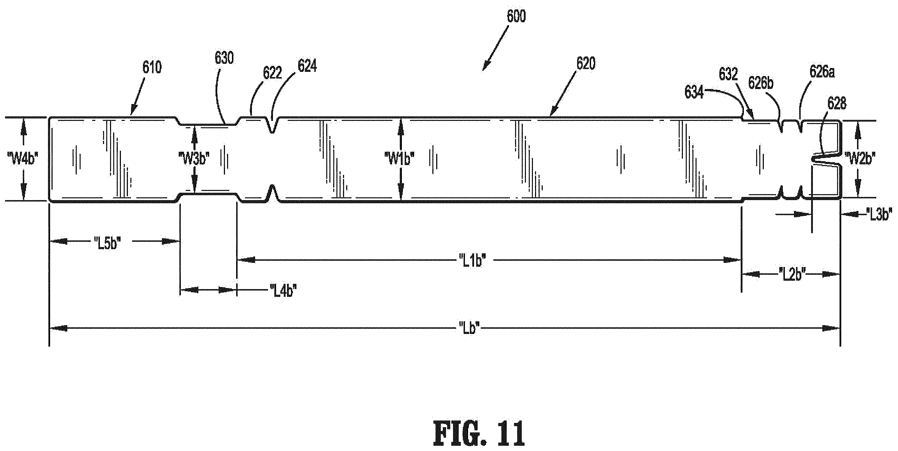

Turning now to FIG. 11, a surgical buttress having a uniform profile, in accordance with another embodiment of the present disclosure, is generally illustrated as 600. Buttress 600 includes a head portion 610, a body portion 620, a neck portion 630 interconnecting head portion 610 and body portion 620, and a tail portion 632 extending proximally from body portion 620.

Buttress 600, similar to buttress 500, is configured to be detachably secured to any sized anvil assembly 300 and/or cartridge assembly 200, as described above. Body portion 620 of buttress 600 defines a pair of opposing distal recesses 624 formed in opposed transverse side edges near a distal location 622 thereof.

In order to accommodate various types of profiles, tail portion 632 of buttress 600 includes two pairs of opposing recesses, a first proximal pair of recesses 626a, and a second proximal pair of recesses 626b (located distal of the first proximal pair of recesses 626a). Each of the proximal pair of recesses 626a, 626b has a substantially v-shape profile. Each of the proximal pair of recesses 626a, 626b is shallower as compared to the proximal pair of recesses 526a, 526b of tail portion 532 of buttress 500.

Neck portion 630 connects head portion 610 to a distal end of body portion 620. Generally, head portion 610 is in a substantially rectangular shape which is used as a tab to facilitate placement of buttress 600 in position on anvil assembly 300 and/or cartridge assembly 200. Following placement of buttress 600 on anvil assembly 300 and/or cartridge assembly 200, under at least certain circumstances, head portion 610 and neck portion 630 may be torn, damaged, or otherwise cut away from body portion 620.

Tail portion 632 of buttress 600 includes a notch 628 at a proximal edge thereof. Notch 628 is substantially centered with respect to the longitudinal axis. Notch 628 which has a U shaped configuration, a triangular, or V-shape configuration, and may be utilized to provide a lead-in for the knife during a cutting of buttress 600. Notch 628 has a length "L3b", as seen in FIG. 11. Length "L3b" of notch 628 of buttress 600 is greater than length "L3a" of notch 528 of buttress 500, and the notch 628 ends just before the first recesses 626a.

As seen in FIG. 11, buttress 600 has an overall length "Lb", which is greater than the overall length "La" of buttress 500. Body portion 620 of buttress 600 has a length "L1b", and tail portion 632 has a length "L2b". Body portion 620 has a width "W1b" and tail portion 632 has a width "W2b" which is less that width "W1b" of body portion 620, wherein a shoulder 634 is defined between a side edge of body portion 620 and a side edge of tail portion 632. It is contemplated that a shoulder 634 is provided or defined along each opposed side edge of buttress 600.

With reference to FIGS. 3 and 11, it is contemplated that body portion 620 of buttress 600 has a length "L1b" which is greater than length "L1a" of body portion 520 of buttress 500. Additionally, it is contemplated that tail portion 632 of buttress 600 has a length "L2b" which is greater than length "L2a" of tail portion 532 of buttress 500.

With reference back to FIG. 11, neck portion 630 of buttress 600 has a length "L4b", and head portion 610 has a length "L5b". Neck portion 630 of buttress 600 has a width "W3b" which is less that width "W1b" of body portion 620, and which is substantially equal to width "W2b" of tail portion 632. Also, head portion 610 has a width "W4b" which is substantially equal to width "W1b" of body portion 620.

With reference to FIGS. 3 and 11, it is contemplated that neck portion 630 of buttress 600 has a width "W3b" which is greater than width "W3a" of neck portion 530 of buttress 500. Additionally, it is contemplated that head portion 610 of buttress 600 has a length "L5b" which is greater than length "L5a" of head portion 510 of buttress 500.

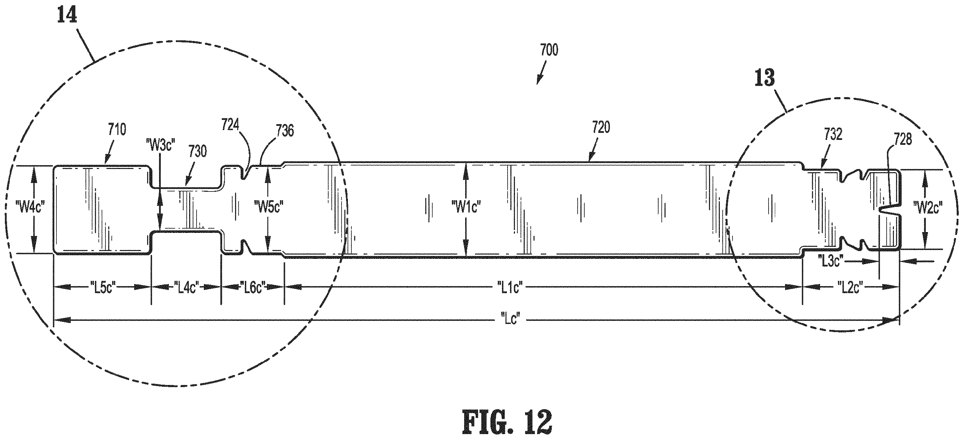

Turning now to FIGS. 12-14, a surgical buttress having a uniform profile, in accordance with yet another embodiment of the present disclosure, is generally illustrated as 700. Buttress 700 includes a head portion 710, a body portion 720, a nose portion 736 extending distally from body portion 720, a neck portion 730 interconnecting nose portion 736 and body portion 720, and a tail portion 732 extending proximally from body portion 720.

As illustrated in FIGS. 12-14, buttress 700 has an overall length "Lc", which is greater than the overall length "La" of buttress 500. Body portion 720 of buttress 700 has a length "L1c", tail portion 732 has a length "L2c", and nose portion 734 has a length "L6c". Body portion 720 has a width "W1c", tail portion 732 has a width "W2c" which is less that width "W1c" of body portion 720, wherein a shoulder is defined between a side edge of body portion 720 and a side edge of tail portion 732, and nose portion 734 has a width "W5c" which is less that width "W1c" of body portion 720, wherein a shoulder is defined between a side edge of body portion 720 and a side edge of nose portion 734. It is contemplated that shoulders are provided or defined along each opposed side edge of buttress 700.

With reference to FIGS. 3, 12 and 13, it is contemplated that body portion 720 of buttress 700 has a length "L1c" which is greater than length "L1a" of body portion 520 of buttress 500. Additionally, it is contemplated that tail portion 732 of buttress 700 has a length "L2c" which is greater than length "L2a" of tail portion 532 of buttress 500.

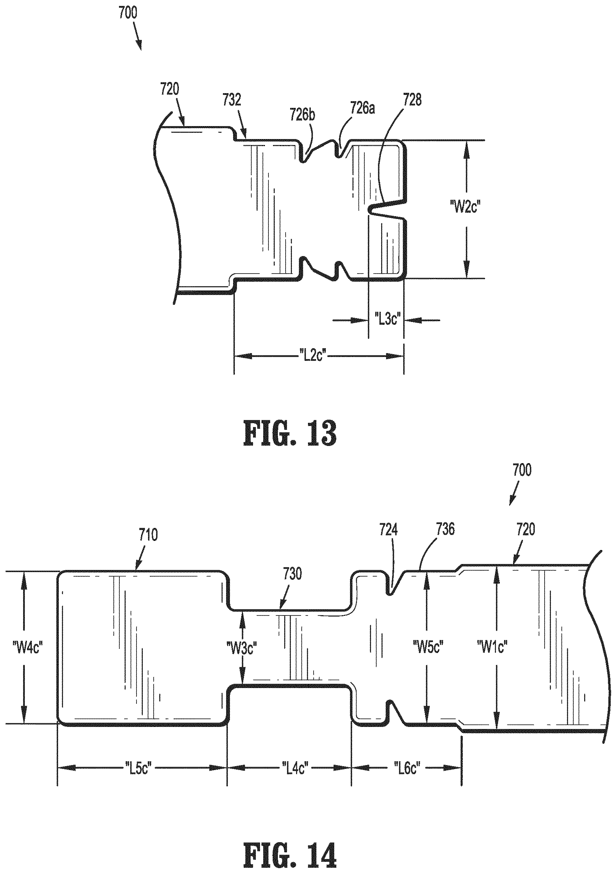

With reference back to FIGS. 12 and 14, nose portion 736 of buttress 700 has a length "L6c."

Neck portion 730 of buttress 700 has a length "L4c", and head portion 710 of buttress 700 has a length "L5c". Neck portion 730 of buttress 700 has a width "W3c" which is less that width "W5c" of nose portion 736. Head portion 710 of buttress 700 has a width "W4c" which is substantially equal to width "W5c" of nose portion 736.

With reference to FIGS. 12 and 13, it is contemplated that neck portion 730 of buttress 700 has a width "W3c" which is greater than width "W3a" of neck portion 530 of buttress 500. Additionally, it is contemplated that head portion 710 of buttress 700 has a length "L5c" which is substantially equal to length "L5a" of head portion 510 of buttress 500.

Buttress 700, similar to buttress 600, is configured to be detachably secured to any sized anvil assembly 300 and/or cartridge assembly 200, as described above. Buttress 700 defines a pair of opposing distal recesses 724 formed in opposed transverse side edges of nose portion 736. Each distal recess 724 has a substantially v-shaped profile. Specifically, each distal recess 724 includes a distal portion that is oriented orthogonal to a longitudinal axis of buttress 700, and a proximal portion that is oriented transverse to the longitudinal axis of buttress 700. In an embodiment, the proximal portion of each distal recess 724 may be oriented at about a 63° angle relative to the longitudinal axis of buttress 700.

In order to accommodate various types of profiles, tail portion 732 of buttress 700 includes two pairs of opposing recesses, a first proximal pair of recesses 726a, and a second proximal pair of recesses 726b (located distal of the first proximal pair of recesses 726a). Each of the proximal pair of recesses 726a, 726b has a substantially v-shaped profile.

Specifically, each recess of the first proximal pair of recesses 726a and each recess of the second proximal pair of recesses 726b includes a distal portion that is oriented orthogonal to a longitudinal axis of buttress 700, and a proximal portion that is oriented transverse to the longitudinal axis of buttress 700. In an embodiment, the proximal portion of each of the first and second proximal pair of recesses 726a, 726b may be oriented at about a 60° angle relative to the longitudinal axis of buttress 700.

As best illustrated in FIG. 13, the segment or portion of side edges of tail portion 732, located between the first proximal pair of recesses 726a and the second proximal pair of recesses 726b, is angled or tapers towards the longitudinal axis of buttress 700, from a proximal end to a distal end thereof.

With reference to FIGS. 12 and 13, neck portion 730 connects head portion 710 to a distal end of nose portion 734. Generally, head portion 710 is in a substantially rectangular shape which is used as a tab to facilitate placement of buttress 700 in position on anvil assembly 300 and/or cartridge assembly 200. Following placement of buttress 700 on anvil assembly 700 and/or cartridge assembly 200, under at least certain circumstances, head portion 710 and neck portion 730 may be torn, damaged, or otherwise cut away from nose portion 734.

Tail portion 732 of buttress 700 includes a notch 728 at a proximal edge thereof. Notch 728 is substantially centered with respect to the longitudinal axis of buttress 700. Notch 728 my have a U shaped configuration, a triangular, or V-shape configuration, and may be utilized to provide a lead-in for the knife during a cutting of buttress 700. Notch 728 has a length "L3c", as seen in FIGS. 12 and 13. Length "L3c" of notch 728 of buttress 700 is greater than length "L3a" of notch 528 of buttress 500, and the notch 728 ends just before the first recesses 726a.

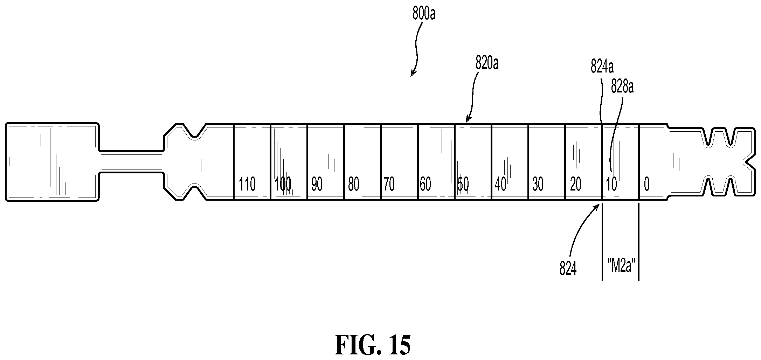

With reference to FIG. 15, a surgical buttress 800a having a uniform profile in accordance with another embodiment of the present disclosure is illustrated. Buttress 800a, similar to buttress 600, is configured to be detachably secured to any sized anvil assembly 300 and/or cartridge assembly 200, as described above.

Surgical buttress 800a includes a body portion 820a having at least one marking 824 disposed therein and/or thereon. Marking(s) 824 include indicia, e.g. patterns, shapes, alphanumeric labeling, etc., to indicate a length of the staple line. As illustrated in FIG. 15, marking(s) 824 may be formed of biocompatible or bioabsorable ink, and/or radiopaque ink or material, that is imprinted longitudinally along the body portion 820a as lines 824a and/or numerals 828a. Line marking(s) 824a may be oriented orthogonal to the longitudinal axis of the buttress 800a and may extend across an entire width of the body portion 820a. Additionally, it is contemplated that a plurality of line markings 824a may be equidistant to one another defining a distance "M2a" between adjacent line markings 824a. It is contemplated that the markings can be formed using radiopaque inks and/or materials.

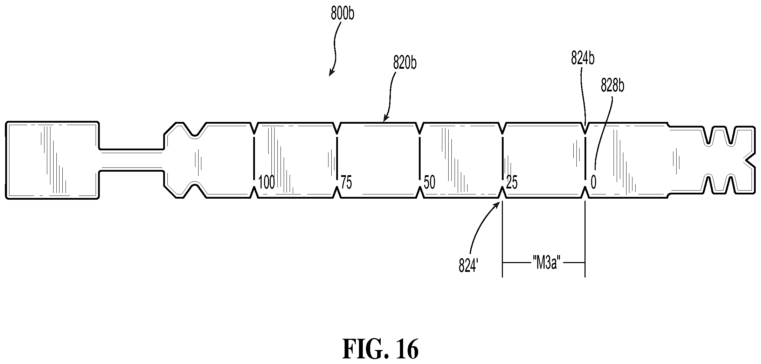

Referring now to FIG. 16, a surgical buttress 800b having a uniform profile in accordance with another embodiment of the present disclosure is illustrated. Buttress 800b, similar to buttress 800a, is configured to be detachably secured to any sized anvil assembly 300 and/or cartridge assembly 200, as described above.

Surgical buttress 800b includes a body portion 820b having at least one marking 824' disposed therein. Marking(s) 824' include indicia and/or recesses, score lines, embossing, etc., to indicate a length of the staple line. As illustrated in FIG. 16, marking(s) 824' may be formed of biocompatible or bioabsorable ink, and/or radiopaque ink, as numerals 828b and as a series of recesses or notches 824b extending longitudinally along the body portion 820b. Recesses 824b may be V-shaped and disposed, one each, on opposing sides of the body portion 820b. Additionally, it is contemplated that a plurality of recesses 824b may be equidistant to one another defining a distance "M3a" between adjacent recesses 824b.

It is contemplated that, in any of the embodiments disclosed herein, markings such as those disclosed herein can be pre-incorporated in a surgical buttress to identify tissue or specimen structures after surgical resection with a surgical instrument or surgical stapler. The markings can be applied during the manufacturing process by methods including thermal or laser processes, printing, sewing, stamping, cutting, dyeing, etc. Alternatively, the markings can be placed by forming notches or holes. Any inks or sewn structures must be biocompatible materials and would desirably be bioabsorbable. It is contemplated that the markings can be used to identify the margins of diseased tissue. For example, the buttress material will be present on the tissue remnant and the specimen removed. A pathologist can communicate to a surgeon the location of any tissue having anomalies with reference to the markings.

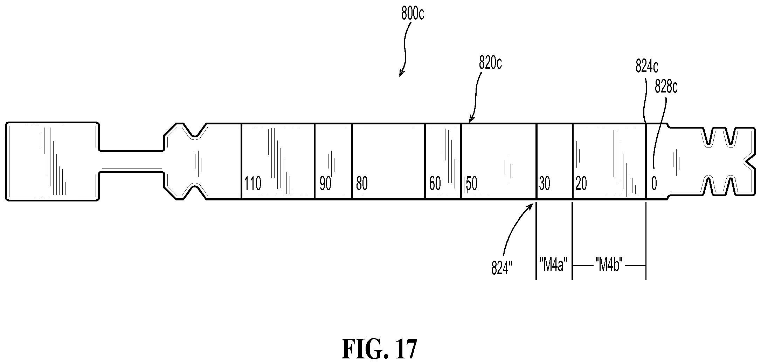

With reference to FIG. 17, a surgical buttress 800c having a uniform profile in accordance with another embodiment of the present disclosure is illustrated. Buttress 800c, similar to buttress 800a, is configured to be detachably secured to any sized anvil assembly 300 and/or cartridge assembly 200, as described above. The buttress 800c can be provided separately and attached to a surgical instrument by the user, or it can be pre-loaded on an instrument, as discussed above.

Surgical buttress 800c includes a body portion 820c having at least one marking 824" disposed therein and/or thereon. Marking(s) 824" include indicia, e.g. patterns, shapes, alphanumeric labeling, etc., to indicate a length of the staple line. As illustrated in FIG. 17, marking(s) 824" may be formed of biocompatible or bioabsorable ink that is imprinted longitudinally along the body portion 820c as lines 824c and/or numerals 828c. Line marking(s) 824c may be oriented orthogonal to the longitudinal axis of buttress 800c and may extend across an entire width of the body portion 820c. Additionally, it is contemplated that a plurality of line markings 824c may not be equidistant to one another defining, for example, a distance "M4a" between a first line marking 824c₁ and a second line marking 824c₂ and a distance "M4b" between a second line marking 824c₂ and an adjacent line marking or third line marking 824c₃.

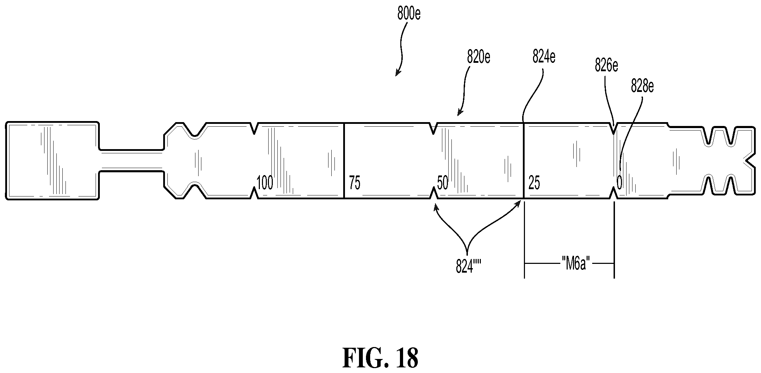

Referring now to FIG. 18, a surgical buttress 800e having a uniform profile in accordance with another embodiment of the present disclosure is illustrated. Buttress 800e, similar to buttress 800a, is configured to be detachably secured to any sized anvil assembly 300 and/or cartridge assembly 200, as described above.

Surgical buttress 800e includes a body portion 820e having at least one marking 824"" disposed therein. Marking(s) 824"" include indicia and/or recesses, score lines, embossing, etc., to indicate a length of the staple line. As illustrated in FIG. 18, marking(s) 824"" may be formed of biocompatible or bioabsorable ink that is imprinted longitudinally along the body portion 820e as lines 824e and/or numerals 828e. The markings(s) 824"" may further include a series of recesses 826e extending longitudinally along the body portion 820e. Recesses 826e may be V-shaped and disposed, one each, on opposing sides of the body portion 820e. Additionally, it is contemplated that a plurality of recesses 826e and line markings 824e are positioned in an alternating pattern and may be equidistant to one another defining a distance "M6a" between the line marking 824e and adjacent recess 826e.

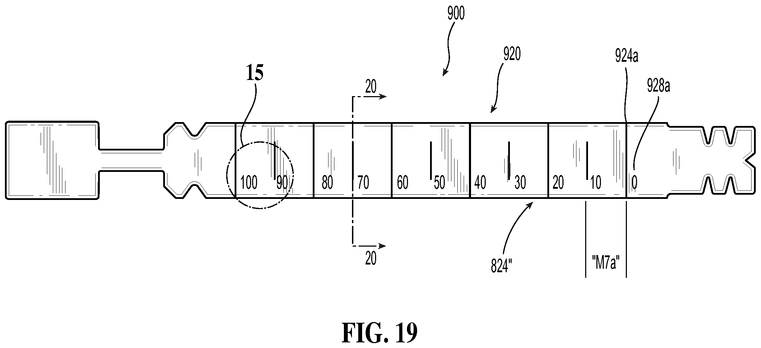

Now turning to FIG. 19, a surgical buttress 900 having a uniform profile in accordance with another embodiment of the present disclosure is illustrated. Buttress 900, similar to buttress 800a, is configured to be detachably secured to any sized anvil assembly 300 and/or cartridge assembly 200, as described above.

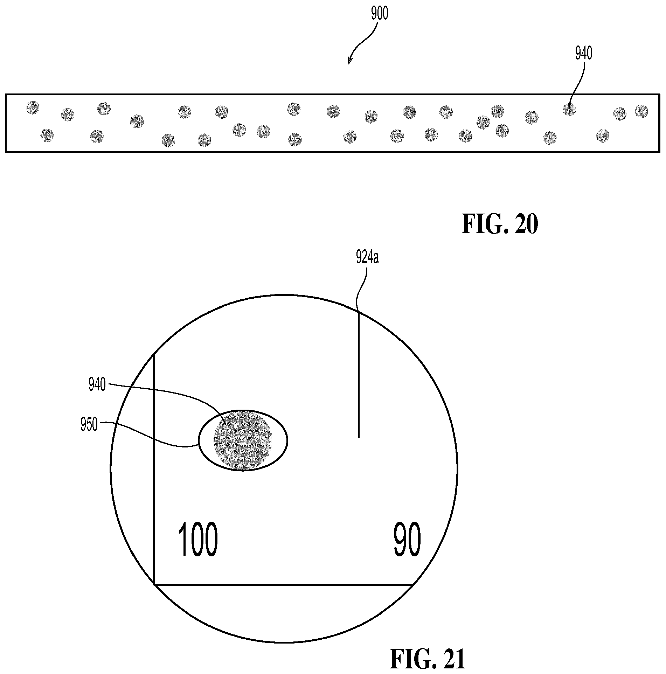

Surgical buttress 900 includes a body portion 920 having at least one marking 924 disposed therein and/or thereon. Marking(s) 924 include indicia, e.g. patterns, shapes, alphanumeric labeling, etc., to indicate a length of the staple line. As illustrated in FIG. 20, marking(s) 924 may be formed of biocompatible or bioabsorable ink that is imprinted longitudinally along the body portion 920 as lines 924 and/or numerals 928. Line marking(s) 928 may be oriented orthogonal to the longitudinal axis of buttress 900 and may vary in length thereby extending across a percentage or an entire width of the body portion 920. Additionally, it is contemplated that a plurality of line markings 924 may be equidistant to one another defining a distance "M6a" between adjacent line markings 924. It is contemplated that any of the embodiments disclosed herein can include the markings disclosed.

In any of the embodiments disclosed herein, the surgical buttress can further include at least one source of radioactive material disposed in or on the body portion of the buttress.

The radioactive material 940 is disposed in and/or on the body portion 920 and includes encapsulated radioactive isotopes. It is envisioned that the radioactive material 940 is formed as brachytherapy seeds and the seeds may be embedded into the body portion 920 of the surgical buttress 900 prior to loading the surgical buttress 900 into the anvil assembly 300 and/or cartridge assembly 200. Radioactive material 940 may take the form of individual radioactive seeds/capsules or radioactive seeds/capsules incorporated into a biocompatible or bioabsorbable thread or the like.

As illustrated in FIG. 20, the radioactive material 940 may be scattered throughout the body portion 920 at varying depths. Alternatively, the radioactive material 940 may be disposed evenly throughout the body portion 920. Additionally, the radioactive material 940 may be deposited on or into a section of the body portion 920. Further, radioactive material 940 may be disposed solely along each lateral side edge of body portion 920 of surgical buttress 900. In this manner, radioactive material 940 is not affected, impacted or disturbed by any staples being fired by the stapling apparatus or by the knife blade that is advanced during a firing of the stapling apparatus. It is contemplated that any of the embodiments disclosed herein can include the radioactive material.

It is contemplated that the process of embedding the radioactive material 940 into the surgical buttress 900 includes inserting the radioactive material 940 into a template (not shown) capable of holding the radioactive material 940, then mating or abutting the template with the surgical buttress 900 and embedding the radioactive material 940 into the body portion 920 of the surgical buttress 900.

As illustrated in FIG. 21, the radioactive material 940 may be in the form of a seed embedded into a pore 950 or pocket disposed in the surgical buttress 900. For example, the pore 950 may be formed while mating the template with the surgical buttress 900 prior to embedding the radioactive material 940 therein.

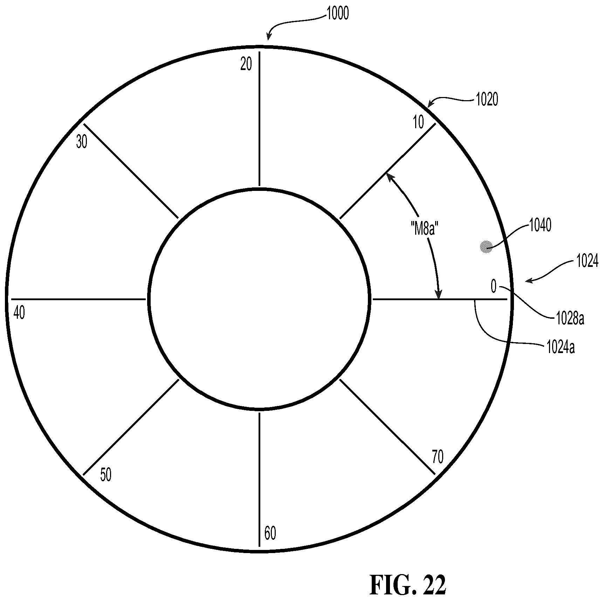

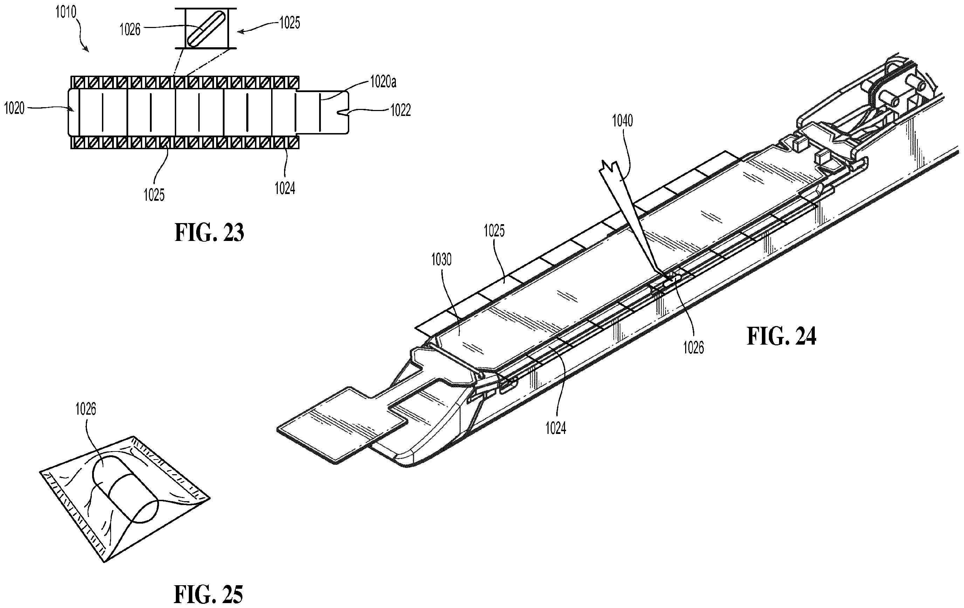

Now turning to FIG. 22, a surgical buttress 1000 having a uniform profile in accordance with another embodiment of the present disclosure is illustrated. Surgical buttress 1000 includes a body portion 1020 that is annular in shape having at least one marking 1024 disposed therein and/or thereon. Marking(s) 1024 include indicia, e.g. patterns, shapes, alphanumeric labeling, etc., to indicate an arc length of the staple line. As illustrated in FIG. 23, marking(s) 1024 may be formed of biocompatible or bioabsorable ink, and/or radiopaque material, that is imprinted radially about the body portion 1020 as lines 1024 and/or numerals 1028. Line marking(s) 1028 may be oriented radial to a central axis of body portion 1020 and may extend across an entire radius of the body portion 1020. Additionally, it is contemplated that a plurality of line markings 1024 may be radially disposed, equidistant to one another, defining an arc length "M7a" between adjacent line markings 1024.

With reference still to FIG. 22, surgical buttress 1000 further includes at least one radioactive material 1040 disposed in and/or on the body portion 1020. It is envisioned that the radioactive material 1040 is formed as brachytherapy seed(s) and the seed(s) may be embedded into the body portion 1020 of the surgical buttress 1000 prior to loading the surgical buttress 1000 into a surgical stapling device.

Additionally, the radioactive material 1040 may be deposited on or into a section of the body portion 1020. It is contemplated that the process of embedding the radioactive material 1040 into the surgical buttress 1000 includes inserting the radioactive material 1040 into a template (not shown) capable of holding the radioactive material 1040, then mating the template with the surgical buttress 1000 and embedding the radioactive material 1040 into the body portion 1020 of the surgical buttress 1000. The radioactive material 1040 may be embedded into a pore or pocket (not shown) disposed in the surgical buttress 1000. For example, the pore or pocket (not shown) may be created while mating the template with the surgical buttress 1000 prior to embedding the radioactive material 1040.

In any of the embodiments disclosed herein, the buttresses may be incorporated into, or configured for use with, devices that are part of a powered surgical system or robotic surgical system.

It is also contemplated that, for any of the buttresses disclosed herein, the buttress can have pre-formed (e.g., by the manufacturer) pockets or pores configured and arranged for the receipt of the radioactive material discussed above. It is contemplated that brachytherapy seeds can be separately packaged and provided, with or without a template, and that the user can apply the seeds to the buttress or buttresses. For example, the user (such as a surgeon or operating room nurse) can place a seed into one or more of the pockets or pores discussed above. It is contemplated that the pockets or pores are pre-formed in the buttress, whether pre-loaded on a surgical instrument or not. In certain embodiments, the pockets or pores are disposed on the lateral edges of the surgical buttress as shown in FIGS. 23-25. The buttress may be configured with lateral wings that extend beyond the sides of the surgical stapler jaws. In any of the embodiments disclosed herein, the buttress material may be porous and configured to encourage tissue ingrowth.

FIG. 23 shows a surgical buttress 1010 having a series of markings, which may be formed as discussed above. The markings 1020a can have a pattern 1020 that would aid a surgeon, operating room nurse, and/or pathologist in identifying the location of certain tissue that was excised from the body. For example, the pattern shown has alternating shorter and longer lines, however, other patterns may be used. The buttress 101 has a central, proximally located notch 1022 at the location where a knife of a surgical stapler will cut through tissue, to aid the advancement of the knife. Such notch 1022 may be triangular or oblong in shape.

The surgical buttress 101 can have wings or lateral edges 1024, along the long sides of the buttress, which extend beyond the sides of the staple cartridge 1030. Such wings 1024 can include or incorporate pockets or pores for the receipt of radioactive material. For example, brachytherapy seeds, which may be configured as discussed herein, can be inserted in the pockets or pores 1025. This can be done by the manufacturer, or by the surgeon or operating room nurse whether or not the staple cartridge assembly has a pre-loaded buttress. A tool 1040, which can be a tweezer or a specially designed device, can be used to safely insert the seed or seeds 1026 into the pockets or pores 1025. One advantage of having a plurality of pockets or pores along the length of the side edges of the buttress 1010 is that the surgeon or operating room nurse can insert seeds 1026 in some or all of the pockets, on one or more sides of the buttress, or may do so in some advantageous pattern.

Although a linear surgical stapling cartridge and buttress is shown, other types are contemplated. For example, a buttress for a circular stapler could have an outer circular edge with a wing or flange that incorporates or includes pockets or pores.

It is contemplated that the radioactive material can come in other forms. For example, the wings, or flanges, can be provided, and a suture or strand having radioactive material inside or on it, can be threaded through the wing or flange. It is contemplated that the radioactive material can be provided as brachytherapy seeds that are installed in pockets that are adhesively attached to the wing or flange of the surgical buttress. It is contemplated that the radioactive material is attached to a surgical buttress at locations other than the lateral sides or outer circular edge.

It is contemplated that a surgical buttress having markings as discussed herein may or may not include the radioactive material or means for attaching radioactive material. It is contemplated that a surgical buttress having the radioactive material or means for attaching radioactive material as discussed herein may or may not include markings.

The present disclosure also includes a surgical kit having a surgical stapling instrument, at least one surgical buttress, and a separate package of encapsulated radioactive material. The surgical stapling instrument can be a circular stapler. The surgical stapling instrument can be a surgical stapling loading unit. The loading unit can be a staple cartridge assembly. The at least one surgical buttress may be pre-loaded onto the surgical stapling instrument. The at least one surgical buttress can include pockets. The at least one surgical buttress can be formed with lateral sides or edges and can include pockets on the lateral sides or edges of the at least one buttress. The lateral sides or edges can include wings that are configured to extend beyond the edges of the cartridge and/or surgical stapling instrument jaws. The encapsulated radioactive material may include radioactive isotopes, such as iodine or cesium isotopes, and may be brachytherapy seeds. The package can include shielding (e.g., for the isotopes). The package can include a template and the isotopes can be attached to the template.

It will be understood that various modifications may be made to the embodiments disclosed herein. For example, the stapling apparatus need not apply staples but rather may apply two part fasteners as is known in the art. Further, the length of the linear row of staples or fasteners may be modified to meet the requirements of a particular surgical procedure. Thus, the length of a single stroke of the actuation shaft and/or the length of the linear row of staples and/or fasteners within a disposable loading unit may be varied accordingly. Therefore, the above description should not be construed as limiting, but merely as exemplifications of preferred embodiments. Those skilled in the art will envision other modifications within the scope of the claims appended thereto.

## Claims

1. A kit for performing surgical stapling, comprising:
a surgical stapling instrument;
at least one surgical buttress (500, 600, 700, 800a, 800b, 800c, 800e, 900, 1000, 1010) having a body portion (520, 620, 720, 820a, 820b, 820c, 820e, 920, 1020 of Fig 22) having lateral sides defining a plurality of pockets (1025); and
a package of capsules of radioactive material (1026) for placement in one or more of the plurality of pockets (1025).

2. The kit of claim 1, wherein the surgical stapling instrument is a surgical stapling loading unit (100).

3. The kit of claim 1 or claim 2, wherein the loading unit (100) is a removable and replaceable staple cartridge assembly (200); and/or wherein the at least one surgical buttress (500, 600, 700, 800a, 800b, 800c, 800e, 900, 1000, 1010) is pre-loaded onto the surgical stapling instrument.

4. The kit of any of claims 1 to 3, wherein lateral sides are wings (1024 of Fig 23/24) that extend beyond the sides of the surgical stapling instrument, the wings incorporating the pockets (1025) for receipt of the capsules of radioactive material (1026).

5. The kit of any of claims 1 to 4, wherein the capsules of radioactive material (1026) are brachytherapy seeds; preferably wherein the brachytherapy seeds have isotopes of cesium or iodine.

6. The kit of any of claims 1 to 5, wherein the package has shielding; preferably wherein the package has a template and the capsules of radioactive material (1026) are attached to the template.

7. A method of embedding radioactive material into a surgical buttress (500, 600, 700, 800a, 800b, 800c, 800e, 900, 1000, 1010), comprising:
providing a surgical buttress (500, 600, 700, 800a, 800b, 800c, 800e, 900, 1000, 1010) having lateral sides defining a plurality of pockets (1025);
providing a package of capsules of radioactive material (1026); and
inserting the capsules of radioactive material (1026) into one or more of the plurality of pockets (1025).

8. The method of claim 7, wherein the capsules of radioactive material (1026) are brachytherapy seeds; and/or wherein the brachytherapy seeds include one of iodine and cesium isotopes.

9. A kit according to claim 1, wherein the body portion (520, 620, 720, 820a, 820b, 820c, 820e, 920, 1020 of Fig 22) includes biocompatible and bioabsorbable materials.

10. A kit according to claim 1, wherein the surgical buttress (500, 600, 700, 800a, 800b, 800c, 800e, 900, 1000, 1010) is formed with at least one marking (824, 924, 1024 of Fig 22, 1020 of Fig 23) provided at least one of in or on the body portion (520, 620, 720, 820a, 820b, 820c, 820e, 920, 1020 of Fig 22).

## Patentansprüche

1. Kit zum Durchführen chirurgischen Klammerns, umfassend:
ein chirurgisches Klammerinstrument;
mindestens eine chirurgische Stütze (500, 600, 700, 800a, 800b, 800c, 800e, 900, 1000, 1010), die einen Körperabschnitt (520, 620, 720, 820a, 820b, 820c, 820e, 920, 1020 der Fig. 22) hat, der laterale Seiten hat, die eine Vielzahl von Taschen (1025) definieren; und
ein Paket von Kapseln mit radioaktivem Material (1026) zur Platzierung in einer oder mehreren der Vielzahl von Taschen (1025).

2. Kit nach Anspruch 1, wobei das chirurgische Klammerinstrument eine chirurgische Klammerladeeinheit (100) ist.

3. Kit nach Anspruch 1 oder Anspruch 2, wobei die Ladeeinheit (100) eine entfernbare und ersetzbare Klammerkartuschenanordnung (200) ist; und/oder wobei die mindestens eine chirurgische Stütze (500, 600, 700, 800a, 800b, 800c, 800e, 900, 1000, 1010) auf dem chirurgischen Klammerinstrument vorinstalliert ist.

4. Kit nach einem der Ansprüche 1 bis 3, wobei laterale Seiten Flügel (1024 der Fig. 23/24) sind, die sich über die Seiten des chirurgischen Klammerinstruments hinaus erstrecken, wobei die Flügel die Taschen (1025) integrieren zum Empfang der Kapseln mit radioaktivem Material (1026).

5. Kit nach einem der Ansprüche 1 bis 4, wobei die Kapseln mit radioaktivem Material (1026) Brachytherapie-Samen sind; vorzugsweise wobei die Brachytherapie-Samen Isotope von Cäsium oder lod haben.

6. Kit nach einem der Ansprüche 1 bis 5, wobei das Paket eine Abschirmung hat; vorzugsweise wobei das Paket eine Vorlage hat und wobei die Kapseln mit radioaktivem Material (1026) an der Vorlage angebracht sind.

7. Verfahren zum Einbetten von radioaktivem Material in eine chirurgische Stütze (500, 600, 700, 800a, 800b, 800c, 800e, 900, 1000, 1010), umfassend:
Bereitstellen einer chirurgischen Stütze (500, 600, 700, 800a, 800b, 800c, 800e, 900, 1000, 1010), die laterale Seiten hat, die eine Vielzahl von Taschen (1025) definieren;
Bereitstellen eines Pakets von Kapseln mit radioaktivem Material (1026); und
Einfügen der Kapseln mit radioaktivem Material (1026) in eine oder mehrere der Vielzahl von Taschen (1025).

8. Verfahren nach Anspruch 7, wobei die Kapseln mit radioaktivem Material (1026) Brachytherapie-Samen sind; und/oder wobei die Brachytherapie-Samen eines aus lod- und Cäsium-Isotopen einschließen.

9. Kit nach Anspruch 1, wobei der Körperabschnitt (520, 620, 720, 820a, 820b, 820c, 820e, 920, 1020 der Fig. 22) biokompatibles und bioabsorbierbares Material einschließt.

10. Kit nach Anspruch 1, wobei die chirurgische Stütze (500, 600, 700, 800a, 800b, 800c, 800e, 900, 1000, 1010) mit mindestens einer Markierung (824, 924, 1024 der Fig. 22, 1020 der Fig. 23) gebildet ist, bereitgestellt mindestens einem aus in oder auf dem Körperabschnitt (520, 620, 720, 820a, 820b, 820c, 820e, 920, 1020 der Fig. 22).

## Revendications

1. Kit de réalisation d'un agrafage chirurgical, comprenant :
un instrument d'agrafage chirurgical;
au moins un contrefort chirurgical (500, 600, 700, 800a, 800b, 800c, 800e, 900, 1000, 1010) ayant une portion de corps (520, 620, 720, 820a, 820b, 820c, 820e, 920, 1020 de la figure 22) ayant des côtés latéraux définissant une pluralité de poches (1025) ; et
un paquet de capsules de matériau radioactif (1026) à placer dans une ou plusieurs de la pluralité de poches (1025).

2. Kit selon la revendication 1, dans lequel l'instrument d'agrafage chirurgical est une unité de charge d'agrafage chirurgical (100).

3. Kit selon la revendication 1 ou la revendication 2, dans lequel l'unité de charge (100) est un ensemble à cartouches d'agrafes amovible et remplaçable (200) ; et/ou dans lequel l'au moins un contrefort chirurgical (500, 600, 700, 800a, 800b, 800c, 800e, 900, 1000, 1010) est préchargé sur l'instrument d'agrafage chirurgical.

4. Kit selon l'une quelconque des revendications 1 à 3, dans lequel des côtés latéraux sont des ailes (1024 des figures 23/24) qui s'étendent au-delà des côtés de l'instrument d'agrafage chirurgical, les ailes incorporant les poches (1025) pour la réception des capsules de matériau radioactif (1026).

5. Kit selon l'une quelconque des revendications 1 à 4, dans lequel les capsules de matériau radioactif (1026) sont des grains de curiethérapie ; de préférence dans lequel les grains de curiethérapie ont des isotopes de césium ou d'iode.

6. Kit selon l'une quelconque des revendications 1 à 5, dans lequel le paquet a un blindage ; de préférence dans lequel le paquet a un gabarit et les capsules de matériau radioactif (1026) sont fixées au gabarit.

7. Procédé d'intégration de matériau radioactif dans un contrefort chirurgical (500, 600, 700, 800a, 800b, 800c, 800e, 900, 1000, 1010), comprenant :
la fourniture d'un contrefort chirurgical (500, 600, 700, 800a, 800b, 800c, 800e, 900, 1000, 1010) ayant des côtés latéraux définissant une pluralité de poches (1025) ;
la fourniture d'un paquet de capsules de matériau radioactif (1026) ; et
l'insertion des capsules de matériau radioactif (1026) dans une ou plusieurs de la pluralité de poches (1025).

8. Procédé selon la revendication 7, dans lequel les capsules de matériau radioactif (1026) sont des grains de curiethérapie ; et/ou dans lequel les grains de curiethérapie incluent l'un d'isotopes d'iode et de césium.

9. Kit selon la revendication 1, dans lequel la portion de corps (520, 620, 720, 820a, 820b, 820c, 820e, 920, 1020 de la figure 22) inclut des matériaux biocompatibles et bio-absorbants.

10. Kit selon la revendication 1, dans lequel le contrefort chirurgical (500, 600, 700, 800a, 800b, 800c, 800e, 900, 1000, 1010) est formé avec au moins un marquage (824, 924, 1024 de la figure 22, 1020 de la figure 23) prévu au moins l'une de dans ou sur la portion de corps (520, 620, 720, 820a, 820b, 820c, 820e, 920, 1020 de la figure 22).
